**Europäisches Patentamt**

**European Patent Office**

**Office européen des brevets**

(11) Veröffentlichungsnummer: **0 073 980**
**B1**

(12) # EUROPÄISCHE PATENTSCHRIFT

(45) Veröffentlichungstag der Patentschrift:
**27.12.85**

(51) Int. Cl.⁴: **G 01 N 21/55, G 01 N 33/543**

(21) Anmeldenummer: **82107564.5**

(22) Anmeldetag: **19.08.82**

(54) Verfahren und Vorrichtung zur Bestimmung biologischer Komponenten.

(30) Priorität: **05.09.81 DE 3135196**

(43) Veröffentlichungstag der Anmeldung:
**16.03.83 Patentblatt 83/11**

(45) Bekanntmachung des Hinweises auf die Patenterteilung:
**27.12.85 Patentblatt 85/52**

(84) Benannte Vertragsstaaten:
**BE CH DE FR GB IT LI NL SE**

(56) Entgegenhaltungen:
**EP - A - 0 019 088**
**DE - A - 2 550 420**
**DE - A - 2 638 250**
**DE - A - 2 756 110**
**US - A - 3 905 767**

(73) Patentinhaber: **Lundström, Kurt Ingemar, Prof. Dr.,
Färgaregatan 10, S-582 52 Linköping (SE)**

(72) Erfinder: **Lundström, Kurt Ingemar, Dr. Prof. Dipl.-Ing.,
Färgaregatan 10, S-582 52 Linköping (SE)**
Erfinder: **Arwin, Hans Rune, Dr. Dipl.-Ing.,
Rydsvägen 132, S-582 48 Linköping (SE)**
Erfinder: **Rieke, Erwin, Dr., Hermannstrasse 12,
D-6104 Seeheim-Jugenheim 1 (DE)**
Erfinder: **Sielaff, Günter, Dr., Schelmengasse 24,
D-6140 Bensheim (DE)**
Erfinder: **Hennrich, Norbert, Dr., Haydnweg 14,
D-6100 Darmstadt (DE)**

(74) Vertreter: **Willquist, Bo Lorentz, PATS Willquist Patenter
S:t. Larsgatan 32 B, S-582 24 Linköping (SE)**

## Beschreibung

Die Erfindung betrifft Verfahren und Vorrichtungen zur quantitativen Bestimmung einer Komponente aus einer Gruppe, bestehend aus spezifisch bindenden Rezeptoren und Substanzen, die von diesen Rezeptoren spezifisch gebunden werden können, mit Hilfe Lichtstrahlung.

Die gebräuchlichsten Verfahren zur empfindlichen immunologischen Bestimmung von Antikörpern, Antigenen und Haptenen basieren auf der Verwendung von Markierungssubstanzen wie Radioisotope, Enzyme oder Fluorochrome, die chemisch an eine der Komponenten gekuppelt werden. Die Notwendigkeit, Antikörper, Antigene oder Haptene mit einer Markierungssubstanz zu kuppeln, beinhaltet jedoch eine Reihe wesentlicher Nachteile: durch die chemische Verknüpfung eines Antikörpers (Antigens) mit z. B. einem Enzym wird der Antikörper (Antigen) in seinem Bindungsverhalten gestört, d. h. die Bindungsaffinität nimmt ab; durch die chemische Verknüpfung eines Enzyms mit einem Antikörper (Antigen) wird die enzymatische Aktivität wesentlich vermindert; die Stabilität der Konjugate ist geringer als die Stabilität der Einzelsubstanzen; die Konjugate stellen sehr heterogene Verbindungen mit breitem Molekulargewichtsspektrum dar, wodurch eine reproduzierbare Herstellung außerordentlich erschwert wird; im Falle der Bindung der Konjugate an eine feste Phase wird die enzymatische Aktivität durch die Diffusion von Substraten und Produkten beeinflußt.

Aufgrund der geschilderten Nachteile besteht ein Bedürfnis nach einfacheren Methoden, die z. B. eine direkte Messung der immunologischen Reaktion zulassen. Es sind zwar schon solche Verfahren bekannt, diese sind jedoch entweder wie die Trübungsmessung nicht empfindlich genug oder, wie die Bestimmung von Antigen-Antikörperschichten auf Metalloberflächen durch Ellipsometrie, zu aufwendig und zu kompliziert.

Eine gegenüber einem konventionellen Ellipsometer etwas vereinfachte Meßanordnung zur Bestimmung von dünnen Schichten wird in der europäischen Patentanmeldung 19 088 beschrieben, wobei im wesentlichen nur der Kompensator eines Ellipsometers durch eine Referenzoberfläche ersetzt ist. In der deutschen Offenlegungsschrift 2 638 250 wird ein einfaches Verfahren zur Erfassung von Antigen-Antikörperschichten auf mit Metallkügelchen beschichteten Gläsern beschrieben, das zwar die Schwierigkeiten der Ellipsometrie umgeht, jedoch nur semiquantitative Aussagen liefert.

Der vorliegenden Erfindung liegt die Aufgabe zugrunde, Verfahren, und Vorrichtungen zur Bestimmung von Antikörpern, Antigenen und Haptenen zur Verfügung zu stellen, mit denen die geschilderten Nachteile vermieden werden können.

Erfindungsgemäß wurde diese Aufgabe dadurch gelöst, daß man eine dieser Komponenten auf einer reflektierenden Oberfläche immobilisiert, mit der zu bestimmenden Komponente inkubiert, bestrahlt und anhand der reflektierten Strahlung die Konzentration ermittelt.

Gegenstand der Erfindung ist ein Verfahren zur quantitativen Bestimmung einer Komponente aus einer Gruppe, bestehend aus spezifisch bindenden Rezeptoren und Substanzen, die von diesen Rezeptoren spezifisch gebunden werden können, mit Hilfe Lichtstrahlung, das dadurch gekennzeichnet ist, daß man eine der Komponenten nach Immobilisierung auf der Oberfläche eines Materials, dessen Brechungsindex hoch ist und sich von dem der zu messenden Komponentenschicht unterscheidet, mit der jeweils anderen zu bestimmenden Komponente inkubiert wonach man gegebenenfalls die gebundene zu bestimmende Komponente nach der Sandwich-Methode mit weiteren Komponenten, die markiert sein können, inkubiert und beschichtet, die Oberfläche mit parallel zur Einfallsebene polarisierter Lichtstrahlung bestrahlt, wobei der Einfallswinkel der Strahlung nahe oder gleich dem Winkel $\Phi$ ist, bei dem die Intensität der von der unbeschichteten oder vorbeschichteten Oberfläche reflektierten Strahlung ein Minimum erreicht, und die Intensität der reflektierten Strahlung als Maß für die zu bestimmende Konzentration ermittelt.

Weiterhin betrifft die Erfindung Vorrichtungen zur Durchführung des Verfahrens, die gekennzeichnet sind durch einen Block (1) zur Aufnahme eines Lichtquellengehäuses (2) und eines Detektorgehäuses (3) wobei sich die optischen Achsen (5) und (6) beider Einheiten auf der zu untersuchenden Oberfläche des Materials (4) unter dem Winkel $\Phi$ zur Oberflächennormalen schneiden und im Lichtquellengehäuse (2) oder im Detektorgehäuse (3) ein Polarisationsfilter (7) angeordnet ist, das nur den parallel zur Einfallsebene polarisierten Teil der Strahlung der Auswertung zuführt.

Überraschenderweise hat sich gezeigt, daß mit dem erfindungsgemäßen Verfahren ein sehr einfacher quantitativer Nachweis immunologischer Reaktionen gefunden wurde, indem die Ermittlung der ellipsometrischen Winkel entfällt und durch eine rein photometrische Detektion ersetzt wird, bei der weder Manipulationen am Gerät, wie Änderungen des Einfallswinkels oder Rotationen der Polarisatoren bzw. des Kompensators, noch aufwendige Berechnungen zur Bestimmung der gesuchten Konzentration notwendig sind. Das Verfahren ist mit geringem technischen Aufwand durchführbar, die erforderlichen Vorrichtungen sind ganz wesentlich kleiner, kostengünstiger, störungsunanfälliger und einfacher zu bedienen, als bisher bekannte ellipsometrische Vorrichtungen, und es liefert für inkubierte Plättchen oder Scheibchen in kürzester Zeit konzentrationsproportionale Meßwerte, wodurch es sich auch gut zur Automatisierung sowie zur Simultanbestimmung mehrerer immunologischer Parameter eignet.

Voraussetzung für das erfindungsgemäße Verfahren ist, daß der Brechungsindex des Materials, auf dessen Oberfläche das Antigen/ Hapten oder der Antikörper immobilisiert werden soll, sich deutlich vom Brechungsindex der zu messenden Antigen/Hapten-Antikörperschicht unterscheidet und ein ausgeprägtes Minimum in der Darstellung des Reflektionskoeffizienten für parallel zur Einfallsebene polarisierte Strahlung gegen den Einfallswinkel $\Phi$ aufweist.

Bei diesem Einfallswinkel wird vorzugsweise monochromatisches Licht auf die mit der Antigen/Hapten-Anntikörperschicht bedeckte Oberfläche gestrahlt; die Intensität des von der Oberfläche reflektierten Lichts, und zwar des Teils, der parallel zur Einfallsebene polarisiert ist, wird gemessen.

Geeignete Materialien für die Immobilisierung der Komponenten sind solche, die eine ebene reflektierende Oberfläche aufweisen und deren Brechungsindex größer als 1,5, vorzugsweise größer als 2 ist. Das Material kann für die verwendete Strahlung undurchlässig oder durchlässig sein. Es können dafür vor allem Halbleiter, Dielektrika, Metalle oder damit überzogene Träger verwendet werden. Bevorzugte Materialien sind Halbleiter wie Silizium oder Germanium, Gläser, Kunststoffe, z. B. Polymethylmethacrylat, Polystyrol, insbesondere mit Silizium überzogene Träger aus Glas oder Kunststoff sowie beliebige Träger, die mit einer Metallschicht überzogen sind.

Die Oberfläche des gewählten Materials kann vor der Immobilisierung einer der Komponenten vorbeschichtet sein. So ist es z. B. möglich, die Oberfläche mit einem dünnen Polymerfilm zu überziehen, der sich gut für die anschließende Immobilisierung einer der Komponenten eignet. Weiterhin ist es möglich, z. B. bei Verwendung von Silizium oder Germanium, eine dünne Oxidschicht auf der Oberfläche aufzubringen. An dieser Oxidschicht kann dann eine der Komponenten gegebenenfalls nach chemischer Aktivierung des Oxids, in an sich bekannter Weise immobilisiert werden. Andererseits kann diese Oxidschicht auch mit reaktiven Silanen umgesetzt werden, wobei die Immobilisierung auch durch chemische Umsetzung mit noch reaktiven Gruppen des Silans durchgeführt werden kann. Die auf der Oberfläche des Materials präformierte dünne Schicht führt neben ihrer Eignung für die Immobilisierung auch zu einer Erhöhung der Nachweisempfindlichkeit des erfindungsgemäßen Verfahrens.

Zur Durchführung des Verfahrens wird auf der Oberfläche eines oben beschriebenen Materials eine Antigen/Hapten-Schicht bzw. eine Antikörperschicht immobilisiert. Das geschieht am einfachsten durch Inkubation der vorzugsweise gereinigten Oberfläche des Materials mit einer Lösung der zu bestimmenden Komponente. Wird die so gebildete Schicht in Kontakt mit einer Antikörperlösung bzw. einer Antigen/Haptenlösung gebracht, so bindet die erste Schicht spezifisch einen Teil der Moleküle aus dieser Lösung, so daß sich eine mehr oder weniger ausgeprägte zweite Schicht auf der ersten Schicht ausbildet. Der Grad der Bedeckung in dieser zweiten Schicht hängt von der Konzentration der Moleküle in der jeweiligen Lösung sowie von der Inkubationsdauer ab. Es hat sich überraschenderweise gezeigt, daß bei geeigneter Wahl des Materials für die Immobilisierung der Antigen/Haptenschicht bzw. der Antikörperschicht sowie eines definierten Einfallswinkels der Bedeckungsgrad in der zweiten Schicht durch eine einfache Messung der Intensität des von der Oberfläche reflektierten Lichts gemessen werden kann, vorausgesetzt man verwendet Licht, das vor oder/ und nach der Reflektion parallel zur Einfallsebene polarisiert ist.

Mit dem erfindungsgemäßen Verfahren können prinzipiell alle organischen Substanzen, zu denen ein Bindungspartner existiert, quantitativ bestimmt werden. So lassen sich z. B. in analoger Weise wie im Fall Antikörper-Antigen/ Hapten auch Bestimmungen mit den Systemen Hormon-Hormonrezeptor, Lektin-Saccharid, Lektin-Glycoprotein, Enzym-Enzyminhibitor oder ähnlicher Systeme durchführen.

Neben der Konzentrationsbestimmung einer dieser Komponenten über die Messung der Intensität der von der zweiten Schicht reflektierten Strahlung sind noch andere Varianten des erfindungsgemäßen Verfahrens durchführbar. So kann die Bestimmung von Haptenen dadurch erleichtert werden, daß man das zu bestimmende Hapten mit einem Konjugat aus dem Hapten und einer weiteren immunchemisch indifferenten, großvolumigen Substanz um die Bindung an den Oberflächen gebundener Antikörper kompetitieren läßt. In diesem Fall bewirkt die an die Antikörperschicht gebundene großvolumige Substanz eine Signalerhöhung, wobei die Intensität des reflektierten Lichtes umgekehrt proportional zur Konzentration des Haptens in der Untersuchungslösung ist.

Eine weitere Modifikation besteht darin, daß man das Hapten der Untersuchungslösung mit einer bekannten Menge an Antikörper reagieren läßt und dann den noch nicht im Komplex befindlichen Antikörper mit oberflächengebundenem Hapten umsetzt. Hierbei ist es auch möglich, den eingesetzten Antikörper vorher mit einer großvolumigen Substanz wie Ferritin oder mit Partikeln wie Latex-Partikeln zu markieren, um eine Signalverstärkung zu erhalten.

In einer weiteren Variante kann das erfindungsgemäße Verfahren auch nach der sogenannten Sandwich-Methode durchgeführt werden. Dabei reagiert das Antigen der Untersuchungslösung zunächst mit dem oberflächengebundenen Antikörper. In einer weiteren Reaktion kann die entstandene (partielle) Doppelschicht mit dem gleichen oder einem anderen gegen das Antigen gerichteten Antikörper umgesetzt werden, wobei eine dreifache Schicht erhalten wird. Auch dieser Antikörper kann mit einem großvolumigen Molekül oder einem Partikel markiert sein, wobei die ausgewählten Partikel auch Metallpartikel sein können. Die Möglichkeiten zur Signalverstärkung durch eine oder mehrere Antikörper, die gegen die schon gebundene Komponente gerichtet sind oder durch andere Komponenten, die an die schon gebundene Komponente binden oder von diesen gebunden werden, sind durch die obigen Beispiele nicht begrenzt; es können z. B. auch andere Bindungssysteme wie Avidin-Biotin oder Glycoprotein-Lectin verwendet werden.

Ein Mittel, das bei dem beanspruchten Verfahren verwendet wird, besteht im wesentlichen aus Plättchen oder Scheibchen des genannten Materials, auf dessen Oberfläche eine Schicht aus der einen Komponente immobilisiert ist, sowie Standardlösungen mit bekannten Konzentrationen der anderen Komponente und gegebenenfalls eine Lösung einer der Komponenten, die mit einem dritten voluminösen Molekül konjugiert sein kann. Die Bestandteile liegen vorzugsweise in Form einer Testpackung vor.

Die Vorrichtung, mit der das erfindungsgemäße Verfahren durchgeführt werden kann, zeichnet sich durch einen sehr einfachen und kompakten Aufbau aus; sie wird anhand der Abbildungen 1 bis 3 näher erläutert. Abbildung 1 zeigt die schematische Darstellung einer solchen Vorrichtung, die Abbildungen 2 und 3 zeigen vorteilhafte Ausführungsformen.

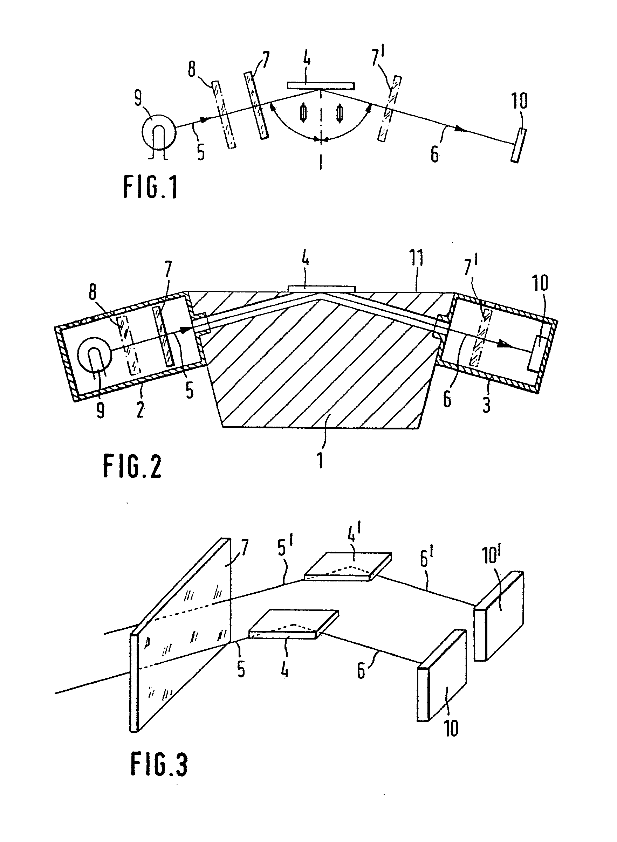

Die in Abbildung 1 dargestellte Vorrichtung besteht aus einer Lichtquelle (9), einem Polarisationsfilter (7) und einem Fotodetektor (10), wobei der Einfallswinkel $\Phi$ und die Durchlaßrichtung des Polarisationsfilters (7) so eingestellt sind, daß die mit dem Detektor (10) nachweisbare Lichtintensität ein Minimum für ein bestimmtes, unbeschichtetes oder vorbeschichtetes Material (4) aufweist. Das Polarisationsfilter (7) kann unter den oben genannten Bedingungen selbstverständlich auch in den Strahlengang (6) anstelle des Strahlengangs (5) gebracht werden; werden 2 Polarisationsfilter (7) und (7') verwendet, so ist die Durchlaßrichtung des Analysators (7') senkrecht zur Einfallsebene zu wählen. Ferner ist es zweckmäßig, wenn auch nicht zwingend, einen Monochromator (8), z. B. ein Filter, in den Strahlengang (5) einzubringen oder einen schmalbandigen Strahler (9) oder einen schmalbandigen Detektor (10) zu verwenden.

Die in Abbildung 2 dargestellte vorteilhafte Ausführungsform der Erfindung besteht aus einem Block (1), der unter dem Einfalls- bzw. Reflektionswinkel $\Phi$ je eine Bohrung zur Aufnahme des Lichtquellengehäuses (2) und des Detektorgehäuses (3) aufweist, wobei die optischen Achsen (5) und (6) sich in der Auflageebene (11) für das Material (4) schneiden. Wird als Material Silizium verwendet, das mit der gewünschten ebenen, hochreflektierenden Oberfläche erhältlich ist, so sind lediglich eine Glühlampe (9), das Polarisationsfilter (7) und ein Bleisulfid-Infrarotdetektor als Beispiel für einen schmalbandigen Fotoempfänger (10) als optische Teile der erfindungsgemäßen Vorrichtung erforderlich. Als Lichtquelle eignen sich auch Laser, Laserdioden usw.

Eine weitere besonders vorteilhafte Ausführungsform ist in Abbildung 3 dargestellt. Der einfache, kompakte Aufbau der erfindungsgemäßen Vorrichtung gestattet es, mit zwei Teilstrahlen (5) und (5') zwei Materialien (4) und (4') bei ein und derselben Ausrichtung des Polarisationsfilters (7) simultan zu bestrahlen und die reflektierten Teilstrahlen (6) und (6') simultan mit 2 Detektoren (10) und (10') nachzuweisen. Hierdurch wird eine Relativmessung zwischen zwei mit unterschiedlichen Rezeptoren beschichteten, sonst aber gleichen Materialien (4) und (4') möglich und somit die Selektivität der Bestimmung erhöht. Weiterhin bietet die erfindungsgemäße Vorrichtung die Möglichkeit, mit einer Vielzahl von Teilstrahlen eine entsprechende Vielzahl von Rezeptor- und Substanz-Gruppen simultan zu bestimmen.

Neben den beschriebenen Ausführungsbeispielen der Vorrichtung sind selbstverständlich weitere, an sich bekannte fotometrische Techniken anwendbar; so lassen sich Relativmessungen auf ein und demselben Materialscheibchen oder Mehrkomponentenbestimmungen auf mehreren Scheibchen auch durch Scannen des Lichtstrahls oder durch Scannen der Materialscheibe(n) ausführen, und es lassen sich die Methoden zur Verbesserung der Signal/Rauschverhältnisse, z. B. Wechsellichtverstärker mit Phasenabstimmung oder Gleichtaktverstärker, die mit der Modulationsfrequenz synchronisiert sind, anwenden.

Ein weiterer Vorteil der erfindungsgemäßen Vorrichtung ist die einfach zu bewerkstelligende Mechanisierbarkeit; durch Verschieben oder Drehen des Materials (4) auf der Auflageebene (11) können mit geringem Aufwand eine hohe Zahl von Materialscheibchen ausgemessen werden, ohne daß aufwendige Justiervorrichtungen erforderlich wären.

Die Erfindung wird durch die folgenden Beispiele näher erläutert.


### Beispiel 1

Bestimmung von anti-Human-Serumalbumin

a) Silanisierung von Siliziumplättchen

Siliziumplättchen ($10 \times 5 \times 0,3$ mm) wurden 10 Minuten in einer Lösung von 10% Dichlordimethylsilan in Trichlorethylen stehen gelassen und anschließend mit Trichlorethylen gewaschen.

b) Beschichtung mit Human-Serumalbumin (HSA)

Die silanisierten Plättchen wurden 30 Minuten in einer 1%igen Lösung von HSA in Saline (0,15 M Natriumchloridlösung) stehen gelassen und anschließend mit destilliertem Wasser gewaschen.

c) Inkubation mit Kaninchen-anti-HSA-Serum (anti-HSA)

Die mit einer Monoschicht an HSA überzogenen Siliziumplättchen wurden 1 Stunde in verschiedene Verdünnungen von Kaninchen-anti-HSA-Serum in Phosphat-gepufferter Saline (pH 7,4) mit 10% normalem Kaninchenserum inkubiert und anschließend mit dest. Wasser gewaschen und im Stickstoffstrom getrocknet.

d) Auswertung

Die Plättchen wurden mit zur Einfallsebene parallel polarisiertem Licht unter einem Einfallswinkel von 76,13° bestrahlt und die Intensität des reflektierten Lichtes wurde mit einem photosensitiven Empfänger gemessen. Wie aus der nachstehenden Tabelle hervorgeht, ist die gemessene Lichtintensität proportional der Konzentration des Antiserums.

| Verdünnung des Antiserums | Meßeinheiten |
| --- | --- |
| 1 : 8 | 130,35 |
| 1 : 16 | 120,67 |
| 1 : 32 | 85,01 |
| 1 : 64 | 60,99 |
| 1 : 128 | 40,28 |
| 1 : 256 | 20,37 |

Beispiel 2

Bestimmung von anti-Human-Fibrinogen

Die Silanisierung der Siliziumplättchen wurde analog Beispiel 1a) durchgeführt. Die Beschichtung mit Human-Fibrinogen wurde wie in Beispiel 1b) beschrieben durchgeführt, wobei statt einer 1%igen HSA-Lösung eine 0,1%ige Fibrinogenlösung verwendet wurde. Die Inkubation mit Ziegen-anti-Human-Fibrinogen erfolgte analog Beispiel 1c), wobei hier Antiserum der Ziege gegen Fibrinogen eingesetzt wurde.

Die Auswertung der Plättchen erfolgte analog Beispiel 1d). Auch hier ist die gemessene Lichtintensität proportional der Konzentration des Antiserums, wie die nachstehende Tabelle zeigt.

| Verdünnung des Antiserums | Meßeinheiten |
| --- | --- |
| 1 : 8 | 201,77 |
| 1 : 16 | 120,03 |
| 1 : 32 | 94,68 |

| Verdünnung des Antiserums | Meßeinheiten |
| --- | --- |
| 1 : 64 | 70,55 |
| 1 : 128 | 59,19 |

Beispiel 3

Bestimmung von anti-Human-Serumalbumin

Zur Aufbringung einer Oxidschicht auf ein Siliziumplättchen wurden Siliziumplättchen zunächst 30 Minuten lang unter strömendem Sauerstoffgas bei 900°C im Ofen stehen gelassen. Danach wurde der Ofen noch 5 Minuten bei 900°C mit Argongas gespült. Die Silanisierung der so behandelten Siliziumplättchen, die Beschichtung mit HSA und die Inkubation mit anti-HSA erfolgte analog Beispiel 1a)—c).

Die Auswertung der Plättchen wurde wie in Beispiel 1d) beschrieben durchgeführt. Aus der nachstehenden Tabelle ist ersichtlich, daß die Aufbringung einer Oxidschicht von ca. 12 nm (120 Å) Dicke mit einer Empfindlichkeitssteigerung verbunden ist.

| Verdünnung des Antiserums | Meßeinheiten |
| --- | --- |
| 1 : 15 | 51,33 |
| 1 : 30 | 32,75 |
| 1 : 60 | 19,61 |
| 1 : 120 | 12,94 |
| 1 : 240 | 9,53 |
| 1 : 480 | 3,11 |

Beispiel 4

Kreuzreaktion mit Fibrinogen-Siliziumplättchen

Die Silanisierung der Siliziumplättchen erfolgte analog Beispiel 1a), und die anschließende Beschichtung mit Fibrinogen analog Beispiel 2b). Zur Untersuchung der Kreuzreaktion von anti-Fibrinogen und anti-HSA wurden die mit Fibrinogen beschichteten Plättchen wie in Beispiel 1c) beschrieben, mit anti-HSA bzw. anti-Fibrinogen inkubiert.

Die Auswertung der Plättchen wurde analog Beispiel 1d) durchgeführt. Wie aus den nachstehenden Tabellen hervorgeht, ist bei den mit anti-HSA inkubierten Plättchen keine Zunahme der Lichtintensität zu verzeichnen.

| Verdünnung des Antiserums (anti-Fibrinogen) | Meßeinheiten |
|---|---|
| 1 : 8 | 75,75 |
| 1 : 16 | 63,18 |
| 1 : 32 | 50,37 |

| Verdünnung des Antiserums (anti-HSA) | Meßeinheiten |
|---|---|
| 1 : 8 | 1,01 |
| 1 : 16 | 0,51 |
| 1 : 32 | 2,34 |

### Beispiel 5

#### Bestimmung von Human-Serumalbumin (HSA) im Sandwich-Verfahren

Die Silanisierung der Siliziumplättchen erfolgte analog Beispiel 1a). Zur Beschichtung mit anti-HSA wurden die silanisierten Plättchen für 2 Stunden in einer Lösung von 100 µg/ml anti-HSA-IgG in Saline stehen gelassen und anschließend mit dest. Wasser gewaschen. Die so beschichteten Plättchen wurden dann 1 Stunde mit verschieden konzentrierten Lösungen von HSA in Phosphatpuffer (pH 7,4) mit 0,1% Rinderserumalbumin stehen gelassen und anschließend mit Phosphatpuffer gewaschen. Die Inkubation mit anti-HSA-Serum wurde wie in Beispiel 1c) durchgeführt, wobei das Antiserum 1 : 8 verdünnt wurde und die Inkubationszeit 3 Stunden betrug.

Die Auswertung der Plättchen wurde wie in Beispiel 1d) durchgeführt. Wie aus der folgenden Tabelle ersichtlich ist, korreliert die gemessene Lichtintensität mit der Menge an HSA in der Inkubationslösung.

| HSA [µg/ml] | Meßeinheiten |
|---|---|
| 2,40 | 142,50 |
| 1,60 | 128,89 |
| 1,20 | 114,31 |
| 1,00 | 98,26 |
| 0,80 | 86,99 |
| 0,60 | 70,02 |
| 0,40 | 46,76 |

| HSA [µg/ml] | Meßeinheiten |
|---|---|
| 0,20 | 24,17 |
| 0,10 | 15,32 |
| 0,05 | 6,68 |

### Beispiel 6

#### Bestimmung von HSA im Sandwich-Verfahren mit einem anti-HSA-Ferritin-Konjugat

Die Silanisierung der Siliziumplättchen, die Beschichtung mit anti-HSA und die Inkubation mit HSA erfolgte analog Beispiel 5. Die Inkubation mit anti-HSA-Ferritin-Konjugat wurde wie in Beispiel 1c) beschrieben durchgeführt, wobei die Lösung des mit Ferritin konjugierten Antikörpers 1 : 10 verdünnt wurde.

Die Auswertung der Plättchen wurde wie in Beispiel 1d) durchgeführt. Wie aus der nachstehenden Tabelle ersichtlich ist, korreliert die gemessene Lichtintensität mit der Menge an HSA in der Inkubationslösung.

| HSA [µg/ml] | Meßeinheiten |
|---|---|
| 1,30 | 202,44 |
| 0,80 | 148,51 |
| 0,40 | 87,97 |
| 0,20 | 50,03 |
| 0,10 | 28,59 |
| 0,05 | 18,18 |
| 0,025 | 7,23 |

### Beispiel 7

#### Bestimmung von Human-Serumalbumin (HSA) im Kompetitionsverfahren

Die Silanisierung der Siliziumplättchen und die Beschichtung mit HSA erfolgte analog Beispiel 1a) und 1b). Zur Inkubation mit HSA und anti-HSA wurde zunächst eine Verdünnungsreihe von Humanseren in Phosphatpuffer, der 1% Rinderserumalbumin enthielt, hergestellt. Zu 1 ml der jeweiligen Verdünnung wurden 0,25 ml anti-HSA (5 mg Antikörper/ml Phosphatpuffer) gegeben und die nach Beispiel 1a) präparierten Plättchen wurden in diese Lösung getaucht. Nach einer Inkubationszeit von 3 Stunden wurden die Plättchen gewaschen und getrocknet.

Die Auswertung der Plättchen wurde wie in Beispiel 1d) durchgeführt. Wie aus der folgenden Tabelle hervorgeht, ist das Meßsignal umgekehrt proportional zur Konzentration des Humanserums. In analoger Weise konnte statt anti-HSA ein anti-HSA-Ferritin-Konjugat eingesetzt werden.

| Verdünnung des Humanserums | Meßeinheiten |
|---|---|
| 1 : 8 | 18,62 |
| 1 : 16 | 41,08 |
| 1 : 32 | 60,00 |
| 1 : 64 | 81,93 |
| 1 : 128 | 109,19 |
| 1 : 256 | 128,94 |

### Beispiel 8

#### Bestimmung von L-Thyroxin im Kompetitinsverfahren

Die Silanisierung der Siliziumplättchen erfolgte analog. Beispiel 1a). Zur Herstellung eines L-Thyroxin-Ferritin-Konjugates wurden 500 mg Ferritin in 250 ml dest. Wasser gelöst und mit 10 mg L-Thyroxin (Natriumsalz) versetzt. Die Lösung wurde bei einem pH-Wert von 5,5 mit 12 mg N-Ethyl-N'-(3-dimethylaminopropyl)-carbodiimid in 5 ml Wasser versetzt und 30 Minuten gerührt. Das Reaktionsprodukt wurde zehnmal gegen 5 Liter Wasser mit 0,9% Benzylalkohol dialysiert. Nach Zentrifugation wurde die Lösung lyophilisiert. Die Plättchen wurden dann analog Beispiel 1b) mit einer Lösung von 100 µg/ml Kaninchen-anti-L-Thyroxin-Antikörpern beschichtet. Zur Inkubation mit L-Thyroxin und L-Thyroxin-Ferritin-Konjugat wurde zunächst eine Verdünnungsreihe (5—100 ng/ml) von L-Thyroxin in Phosphatpuffer mit 1% Rinderserumalbumin (je 1 ml) hergestellt. In diese Lösungen wurden die mit anti-L-Thyroxin beschichteten Plättchen gegeben und 2 Stunden inkubiert. Anschließend wurden 20 µl einer Lösung von L-Thyroxin-Ferritin-Konjugat (1 mg/ml) zugegeben und nochmals 2 Stunden inkubiert. Danach wurde gewaschen und getrocknet.

Die Plättchen wurden wie in Beispiel 1d) beschrieben gemessen. Aus der nachstehenden Tabelle geht hervor, daß die Konzentration an L-Thyroxin umgekehrt proportional der Lichtintensität ist.

| L-Thyroxin [ng/ml] | Meßeinheiten |
|---|---|
| 100 | 5,33 |
| 80 | 6,10 |
| 60 | 8,05 |
| 40 | 10,96 |
| 20 | 14,49 |
| 10 | 17,71 |
| 5 | 19,83 |

### Beispiel 9

#### Bestimmung von Human-Immunoglobulin (h-IgG) im Sandwich-Verfahren mit Immunobeads Kaninchen-anti-human-IgG (a-h-IgG)

Die Silanisierung der Siliziumplättchen erfolgte analog Beispiel 1a). Zur Beschichtung mit Kaninchen-anti-human-IgG wurden die silanisierten Plättchen für 2 Stunden in einer Lösung von 100 µg/ml a-h-IgG in Saline stehen gelassen und anschließend mit dest. Wasser gewaschen. Die so beschichteten Plättchen wurden dann 1 Stunde mit verschieden konzentrierten Lösungen von IgG in Phosphatpuffer (pH 7,4) mit 0,1% Rinderserumalbumin stehen gelassen und anschließend mit Phosphatpuffer gewaschen. Die Inkubation mit Immunobeads Kaninchen-anti-human-IgG wurde wie in Beispiel 1c) durchgeführt, wobei die Immunobeads in einer Konzentration von 1 mg/ml eingesetzt wurden. Es wurde 3 Stunden unter ständigem Schütteln inkubiert.

Die Auswertung erfolgte analog Beispiel 1d). Wie aus der nachstehenden Tabelle ersichtlich ist, korreliert die gemessene Lichtintensität mit der Menge an h-IgG in der Inkubationslösung.

| h-IgG [ng/ml] | Meßeinheiten |
|---|---|
| 80,0 | 172,51 |
| 40,0 | 115,10 |
| 20,0 | 71,82 |
| 10,0 | 42,18 |
| 5,0 | 28,33 |
| 2,5 | 16,71 |
| 1,0 | 8,27 |
| 0,5 | 5,06 |

**Patentansprüche**

1. Verfahren zur quantitativen Bestimmung einer Komponente aus einer Gruppe, bestehend aus spezifisch bindenden Rezeptoren und Substanzen, die von diesen Rezeptoren spezifisch gebunden werden können, mit Hilfe Lichtstrahlung, dadurch gekennzeichnet, daß man eine der Komponenten nach Immobilisierung auf der Oberfläche eines Materials, dessen Brechungsindex hoch ist und sich von dem der zu messenden Komponentenschicht unterscheidet, mit der jeweils anderen zu bestimmenden Komponente inkubiert, wonach man gegebenenfalls die gebundene zu bestimmende Komponente nach der Sandwich-Methode mit weiteren Komponenten, die markiert sein können, inkubiert und beschichtet, die Oberfläche mit parallel zur Einfallsebene polarisierter Lichtstrahlung bestrahlt, wobei der Einfallswinkel der Strahlung nahe oder gleich dem Winkel $\Phi$ ist, bei dem die Intensität der von der unbeschichteten oder vorbeschichteten Oberfläche reflektierten Strahlung ein Minimum erreicht, und die Intensität der reflektierten Strahlung als Maß für die zu bestimmende Konzentration ermittelt.

2. Verfahren nach Anspruch 1, dadurch gekennzeichnet, daß als Material Halbleiter, Dielektrika, Metall oder damit überzogene Träger verwendet werden.

3. Verfahren nach den Ansprüchen 1 bis 2, dadurch gekennzeichnet, daß als Material Silizium oder ein mit Silizium überzogener Träger verwendet wird.

4. Verfahren nach den Ansprüchen 1 bis 3, dadurch gekennzeichnet, daß die Oberfläche des Materials mit einer Oxidschicht, einem Polymerfilm oder durch Silanisierung vorbeschichtet wird.

5. Verfahren nach den Ansprüchen 1 bis 4, dadurch gekennzeichnet, daß mit Hilfe eines zweiten Strahls eine zweite Oberfläche des gleichen, jedoch nicht inkubierten Materials bestrahlt wird und aus der Differenz der beiden reflektierten Intensitäten die zu bestimmende Konzentration ermittelt wird.

6. Verfahren nach den Ansprüchen 1 bis 5, dadurch gekennzeichnet, daß mit Hilfe mehrerer Lichtstrahlen auf mit unterschiedlichen Komponenten beschichtete Materialien eingestrahlt wird und aus den Intensitäten der reflektierten Strahlen die Konzentrationen dieser Komponenten simultan bestimmt werden.

7. Vorrichtung zur Durchführung des Verfahrens nach den Ansprüchen 1 bis 6, gekennzeichnet durch einen Block (1) zur Aufnahme eines Lichtquellengehäuses (2) und eines Detektorgehäuses (3) wobei sich die optischen Achsen (5) und (6) beider Einheiten auf der zu untersuchenden Oberfläche des Materials (4) unter dem Winkel $\Phi$ zur Oberflächennormalen schneiden und im Lichtquellengehäuse (2) oder im Detektorgehäuse (3) ein Polarisationsfilter (7) angeordnet ist, das nur den parallel zur Einfallsebene polarisierten Teil der Strahlung der Auswertung zuführt.

**Claims**

1. A method for the quantitative determination, using light radiation, of a component of an organic system comprising specifically bounding receptor components and substance components which can be specifically bounded by the receptor components, characterized in incubating one of the components, after it has been immobilized on the surface of a material having a high refractive index which is different from that of component layer to be measured, with the other specific components whereafter optionally the bounded component to be determined is incubated and layered according to the sandwich-method with further components which may be marked, irradiating said surface with light radiation which is polarized parallel to the plane of incidence, the angle of incidence of the radiation being approximately equal to or equal to the angle $\Phi$ at which the intensity of the radiation reflected from the unlayered or prelayered surface reaches a minimum, and measuring the intensity of the reflected radiation as a measure of the concentration to be determined.

2. A method according to claim 1, characterized in that semiconductors, dielectrica, metal or carriers coated with it can be used as material.

3. A method according to claims 1—2, characterized in that silicon or a carrier coated with silicon is used as material.

4. A method according to claims 1—3, characterized in that the surface of the material is precoated with an oxide layer, a polymer film or by silanization.

5. A method according to claims 1—4, characterized in that with the aid of a second radiation a second surface of the same material, however not incubated, is radiated and the concentration is determined from the difference between the two reflected intensities.

6. A method according to claims 1—5, characterized in that materials coated with different materials are irradiated with the aid of a multiple of light beams and the concentration of said components are simultaneously determined from the intensities of the reflected beams.

7. Means for carrying out the method according to claims 1—6, characterized in a block (1) accomodating a light source housing (2) and a detector housing (3), the optical axes (5) and (6) of both units intersecting the surface to be investigated of the material (4) at an angle $\Phi$ with respect to the perpendicular to the surface, and the light source housing (2) or the detector housing (3) containing a polarization filter (7) in the optical path which transmits only the portion of the radiation which is polarized parallel to the plane of incidence.

## Revendications

1. Procédé pour doser un composant présent dans un groupe, constitué de récepteurs aptes à former des liaisons spécifiques et de substances qui peuvent se lier de manière spécifique auxdits récepteurs, au moyen d'un rayonnement lumineux, caractérisé en ce que l'on place en incubation l'un des composants après l'avoir immobilisé sur la surface d'un matériau dont l'indice de réfraction est élevé et se distingue de celui de la couche du composant à analyser, avec chacun des autres composants à doser, à la suite de quoi, le cas échéant, on place en incubation et on enduit selon le procédé dit du sandwich avec d'autres composants qui peuvent être marqués, le composant à doser ainsi lié; on soumet la surface à un rayonnement de lumière polarisée parallèlement au plan d'incidence, l'angle d'incidence du rayonnement étant égal à ou proche de l'angle $\Phi$ pour lequel l'intensité du rayonnement réfléchi par la surface non enduite ou portant un revêtement initial, atteint une valeur minimale; et on évalue l'intensité du rayonnement réfléchi, qui sert de mesure de la concentration à déterminer.

2. Procédé selon la revendication 1, caractérisé en ce que le matériau qu'il utilise est un semi-conducteur, un diélectrique, un métal ou un support revêtu de telles substances.

3. Procédé selon la revendication 1 ou 2, caractérisé en ce que le matériau qu'il utilise est du silicium ou un support revêtu de silicium.

4. Procédé selon les revendications 1 à 3, caractérisé en ce que la surface du matériau est enduite d'un revêtement initial constitué par une couche d'oxyde ou par un film de polymère, ou obtenu par une silanisation.

5. Procédé selon les revendications 1 à 4, caractérisé en ce que l'on soumet à un second rayonnement limineux une seconde surface du même matériau, qui n'est toutefois pas incubé, et on détermine la concentration recherchée à partir de la différence des deux intensités réfléchies.

6. Procédé selon les revendications 1 à 5, caractérisé en ce que l'on soumet des matériaux revêtus de composants différents à plusieurs rayonnements lumineux, et on détermine simultanément les concentrations desdits composants à partir des intensités des rayonnements réfléchis.

7. Dispositif pour la mise en oeuvre du procédé selon les revendications 1 à 6, caractérisé en ce qu'il comprend un bloc (1) destiné à recevoir une enceinte d'une source lumineuse (2) et une enceinte d'un détecteur (3), les axes optiques (5) et (6) de ces deux unités se coupant sur la surface à étudier du matériau (4) en formant un angle $\Phi$ par rapport à la normale à cette surface, et en ce que les enceintes de la source lumineuse (2) et du détecteur (3) contiennent un filtre polarisant (7) qui ne laisse passer que la partie du rayonnement d'analyse qui est polarisée parallèlement au plan d'incidence.

FIG.1

FIG.2

FIG.3